# EUROPEAN PATENT APPLICATION

(11) **EP 2 333 576 A2**
(43) Date of publication of application: **15.06.2011**
(21) Application number: 10175433.1
(22) Date of filing: 06.09.2010
(51) Int. Cl.: G01S 7/52, G01S 15/89

(54) **Providing a three-dimensional ultrasound image based on a sub region of interest in an ultrasound system**

(30) Priority: 09.12.2009 KR 20090121600
(71) Applicant: MEDISON CO., LTD., Gangwon-do 250-875 (KR)
(72) Inventor: Lee, Jae Keun, Seoul 135-851 (KR); Kim, Sung Yoon, Seoul 135-851 (KR)
(74) Representative: Schmid, Wolfgang

(57) **Abstract**

Embodiments for providing a three-dimensional (3D) ultrasound image are disclosed. In one embodiment, by way of non-limiting example, an ultrasound system comprises: an ultrasound data acquisition unit configured to transmit and receive ultrasound signals to and from a target object to output a plurality of ultrasound data; a user input unit configured to receive first input information for defining a region of interest (ROI) from a user; and a processing unit in communication with the ultrasound data acquisition unit and the user input unit, the processing unit being configured to form volume data based on the plurality of ultrasound data, define the ROI in the volume data in response to the first input information, and render volume data corresponding to the ROI to form a three-dimensional (3D) ultrasound image, wherein the user input unit is further configured to receive second input information for defining a sub ROI on the 3D ultrasound image, and wherein the processing unit is further configured to define the sub ROI in the volume data based on the second input information, and render volume data corresponding to the sub ROI to form a sub 3D ultrasound image.

## Description

### TECHNICAL FIELD

The present disclosure generally relates to ultrasound imaging, and more particularly to a method of providing a three-dimensional (3D) ultrasound image based on a sub region of interest (ROI) in an ultrasound system.

### BACKGROUND

An ultrasound system has become an important and popular diagnostic tool since it has a wide range of applications. Specifically, due to its non-invasive and non-destructive nature, the ultrasound system has been extensively used in the medical profession. Modem high-performance ultrasound systems and techniques are commonly used to produce two or three-dimensional diagnostic images of internal features of an object (e.g., human organs).

The ultrasound system may provide the three-dimensional ultrasound image including clinical information such as spatial information and anatomical figures of the target object, which cannot be provided by the two-dimensional ultrasound image. The ultrasound system may transmit ultrasound signals into the target object, receive ultrasound echo signals reflected from the target object and form volume data based on the ultrasound echo signals. The ultrasound system may further form the three-dimensional ultrasound image including the clinical information by rendering the volume data.

Conventionally, if a region of interest (ROI) set on a three-dimensional (3D) ultrasound image is changed to observe a specific part of the target object in the 3D ultrasound image, then a new 3D ultrasound image corresponding to the changed ROI is formed. As such, the new 3D ultrasound image is provided in place of the former 3D ultrasound image. It may be necessary to represent the new 3D ultrasound image as well as the former 3D ultrasound image in order to more efficiently observe the target object.

### SUMMARY

Embodiments for providing a plurality of slice images in an ultrasound system are disclosed herein. In one embodiment, by way of non-limiting example, an ultrasound system comprises: an ultrasound data acquisition unit configured to transmit and receive ultrasound signals to and from a target object to output a plurality of ultrasound data; a user input unit configured to receive first input information and second input information from a user; and a processing unit in communication with the ultrasound data acquisition unit and the user input unit, wherein the processing unit is configured to form volume data based on the plurality of ultrasound data, form a plurality of 2D ultrasound images corresponding to a plurality of planes based on the volume data, define a region of interest (ROI) in the volume data in response to the first input information for defining the ROI in the plurality of 2D ultrasound images, render volume data corresponding to the ROI to form a 3D ultrasound image, define a sub ROI in the volume data in response to the second input information for defining the sub ROI in the 3D ultrasound image, and render volume data corresponding to the sub ROI to form a sub 3D ultrasound image.

In another embodiment, there is provided a method of providing a 3D ultrasound image, comprising: a) forming volume data based on a plurality of ultrasound data for a target object; b) forming a plurality of 2D ultrasound images corresponding to a plurality of planes based on the volume data; c) defining a region of interest (ROI) in the volume data in response to first input information for defining the ROI in the plurality of 2D ultrasound images; d) rendering volume data corresponding to the ROI to form a 3D ultrasound image; e) defining a sub ROI in the volume data in response to second input information for defining the sub ROI in the 3D ultrasound image; and f) rendering volume data corresponding to the sub ROI to form a sub 3D ultrasound image.

The Summary is provided to introduce a selection of concepts in a simplified form that are further described below in the Detailed Description. This Summary is not intended to identify key or essential features of the claimed subject matter, nor is it intended to be used in determining the scope of the claimed subject matter.

### BRIEF DESCRIPTION OF THE DRAWINGS

- FIG. 1: is a block diagram showing an illustrative embodiment of an ultrasound system.
- FIG. 2: is a block diagram showing an illustrative embodiment of an ultrasound data acquisition unit.
- FIG. 3: is a schematic diagram showing an example of acquiring ultrasound data corresponding to a plurality of frames.
- FIG. 4: is a flow chart showing a process of forming a three-dimensional (3D) ultrasound image based on a sub region of interest (ROI).
- FIG. 5: is a schematic diagram showing an example of volume data.
- FIG. 6: is a schematic diagram showing an example of 2D ultrasound images, an ROI and a 3D ultrasound image.
- FIG. 7: is a schematic diagram showing an example of an observation position and a sub ROI.
- FIG. 8: is a schematic diagram showing an example of the sub ROI.
- FIG. 9: is a schematic diagram showing an example of the sub ROI.

### DETAILED DESCRIPTION

A detailed description may be provided with reference to the accompanying drawings. One of ordinary skill in the art may realize that the following description is illustrative only and is not in any way limiting.

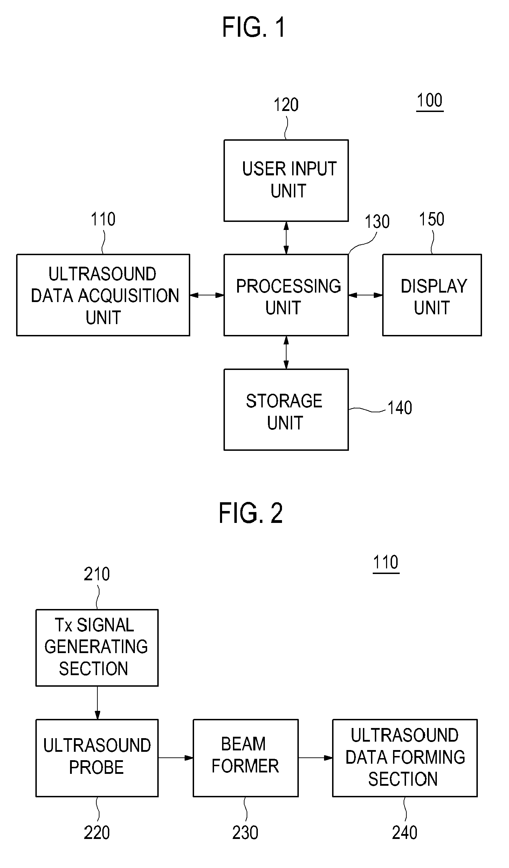

Referring to FIG. 1, an ultrasound system 100 in accordance with an illustrative embodiment is shown. As depicted therein, the ultrasound system 100 may include an ultrasound data acquisition unit 110. The ultrasound data acquisition unit 110 may be configured to transmit and receive ultrasound signals to and from a target object to thereby output ultrasound data.

FIG. 2 is a block diagram showing an illustrative embodiment of the ultrasound data acquisition unit 110. Referring to FIG. 2, the ultrasound data acquisition unit 110 may include a transmit (Tx) signal generating section 210, an ultrasound probe 220, a beam former 230 and an ultrasound data forming section 240.

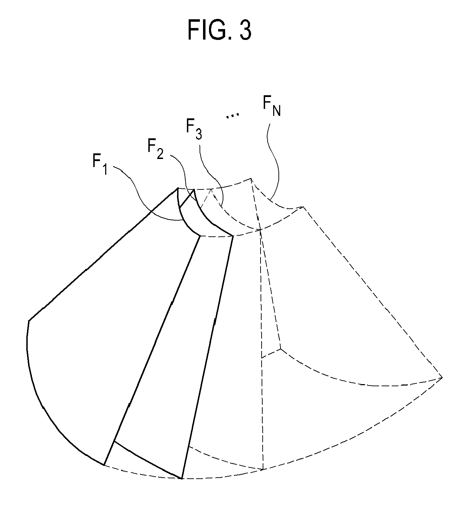

The Tx signal generating section 210 may be configured to generate Tx signals. The Tx signal generating section 210 may generate the Tx signals at every predetermined time to thereby form a plurality of Tx signals corresponding to a plurality of frames Fᵢ (1≤ i ≤ N) representing the target object, as shown in FIG. 3. The frame may include a brightness mode (B mode) image. However, it should be noted herein that the frame may not be limited thereto.

FIG. 3 is a schematic diagram showing an example of acquiring ultrasound data corresponding to the plurality of frames Fᵢ (1≤ i ≤ N). The plurality of frames Fᵢ (1≤ i ≤ N) may represent sectional planes of the target object (not shown).

Referring back to FIG. 2, the ultrasound probe 220 may include a plurality of elements (not shown) for reciprocally converting between ultrasound signals and electrical signals. The ultrasound probe 220 may be configured to transmit ultrasound signals to the target object in response to the Tx signals provided from the Tx signal generating section 210. The ultrasound probe 220 may further receive ultrasound echo signals reflected from the target object to thereby output the received signals. The received signals may be analog signals. The ultrasound probe 220 may include a three-dimensional (3D) mechanical probe, a two-dimensional (2D) array probe and the like. However, it should be noted herein that the ultrasound probe 220 may not be limited thereto.

The beam former 230 may be configured to convert the received signals provided from the ultrasound probe 220 into digital signals. The beam former 230 may further apply delays to the digital signals in consideration of distances between the elements and focal points to thereby output digital receive-focused signals.

The ultrasound data forming section 240 may be configured to form ultrasound data corresponding to each of the plurality of frames Fᵢ (1≤ i ≤ N) based on the digital receive-focused signals provided from the beam former 230. The ultrasound data may be radio frequency (RF) data. However, it should be noted herein that the ultrasound data may not be limited thereto. The ultrasound data forming section 240 may further perform various signal processing (e.g., gain adjustment) to the digital receive-focused signals.

Referring back to FIG. 1, the ultrasound system 100 may further include a user input unit 120. The user input unit 120 may be configured to receive input information from a user. In one embodiment, the input information may include first input information for defining a region of interest (ROI) for obtaining a 3D ultrasound image, as well as second input information for defining a sub ROI in the 3D ultrasound image. The sub ROI will be described below in detail. The input information may further include third input information for performing an image processing upon the 3D ultrasound image. The user input unit 120 may include a control panel, a mouse, a keyboard and the like. However, it should be noted herein that the user input unit 120 may not be limited thereto.

As shown in FIG. 1, the ultrasound system 100 may further include a processing unit 130 in communication with the ultrasound data acquisition unit 110 and the user input unit 120.

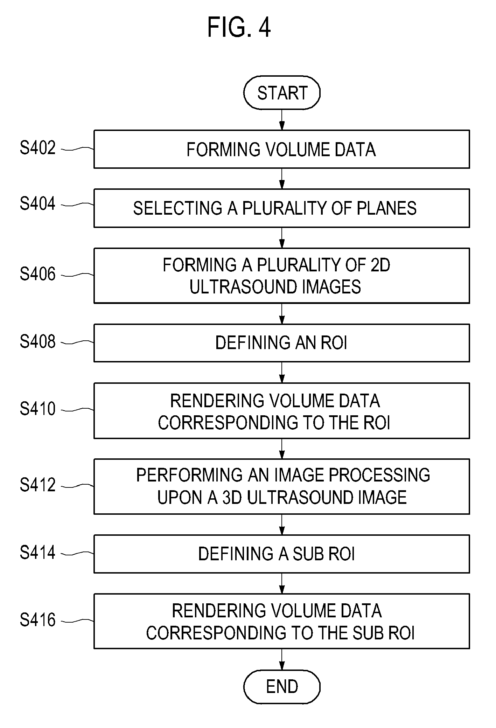

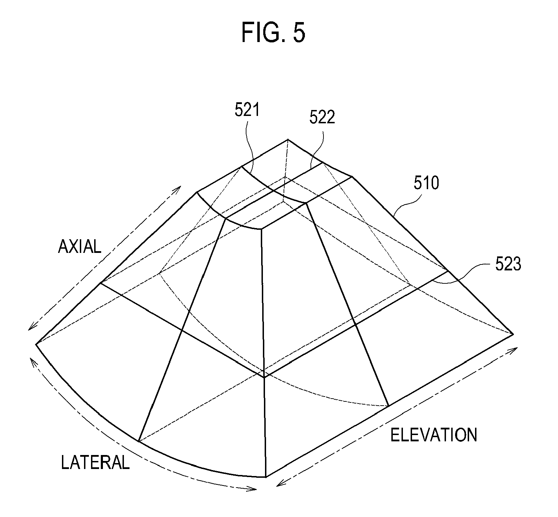

FIG. 4 is a flow chart showing a process of forming a 3D ultrasound image based on the sub ROI. The processing unit 130 may be configured to synthesize the plurality of ultrasound data corresponding to the plurality of frames Fᵢ (1≤ i ≤ N) to thereby form volume data 510 as shown in FIG. 5, at step S402. The volume data 510 may be stored in a storage unit 140 as shown in FIG. 1.

FIG. 5 is a schematic diagram showing an example of the volume data 510. The volume data 510 may include a plurality of voxels (not shown) having brightness values. In FIG. 5, reference numerals 521 to 523 represent an A plane, a B plane and a C plane. The A plane 521, the B plane 522 and the C plane 523 may be mutually orthogonal. Also, in FIG. 5, the axial direction may be a Tx direction of the ultrasound signals, the lateral direction may be a longitudinal direction of the elements, and the elevation direction may be a swing direction of the elements, i.e., a depth direction of a 3D ultrasound image.

The processing unit 130 may be configured to select a plurality of planes from the volume data, at step S404 in FIG. 4. The plurality of planes may be mutually orthogonal. In one embodiment, the plurality of planes may include the A plane 521, the B plane 522 and the C plane 523, as shown in FIG. 5. However, it should be noted herein that the plurality of planes may not be limited thereto.

The processing unit 130 may be configured to form a plurality of 2D ultrasound images corresponding to the plurality of planes based on the volume data, at step S406 in FIG. 4. The plurality of 2D ultrasound images may be displayed on a display unit 150, as shown in FIG. 1. Thus, a user may define the ROI in the plurality of 2D ultrasound images. The 2D ultrasound image may include the B mode image. However, it should be noted herein that the 2D ultrasound image may not be limited thereto.

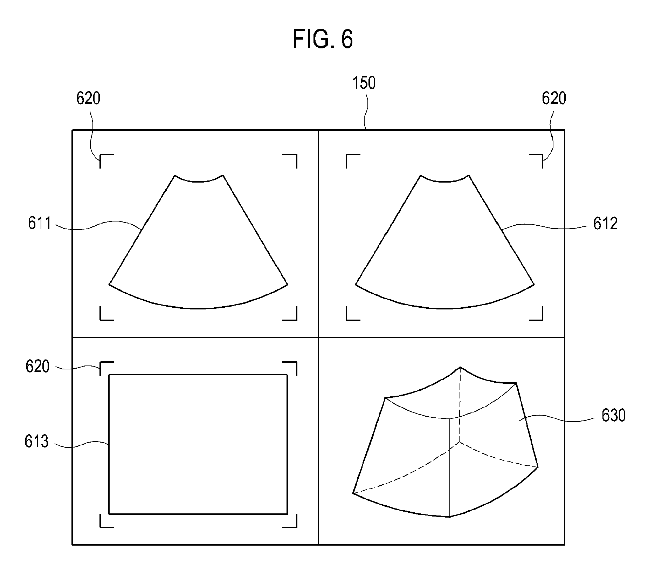

FIG. 6 is a schematic diagram showing an example of the 2D ultrasound images, the ROI and the 3D ultrasound image. As one example, the processing unit 130 may be configured to select the A plane 521 to the C plane 523 from the volume data 510, and form the 2D ultrasound images 611 to 613 corresponding to the A plane 521 to the C plane 523 as shown in FIG. 6.

The processing unit 130 may be configured to define the ROI in the plurality of 2D ultrasound images in response to the input information (i.e., first input information) provided from the user input unit 120, at step S408 in FIG. 4. As one example, the processing unit 130 may define the ROI 620 in the 2D ultrasound images 611 to 613 based on the input information, as shown in FIG. 6.

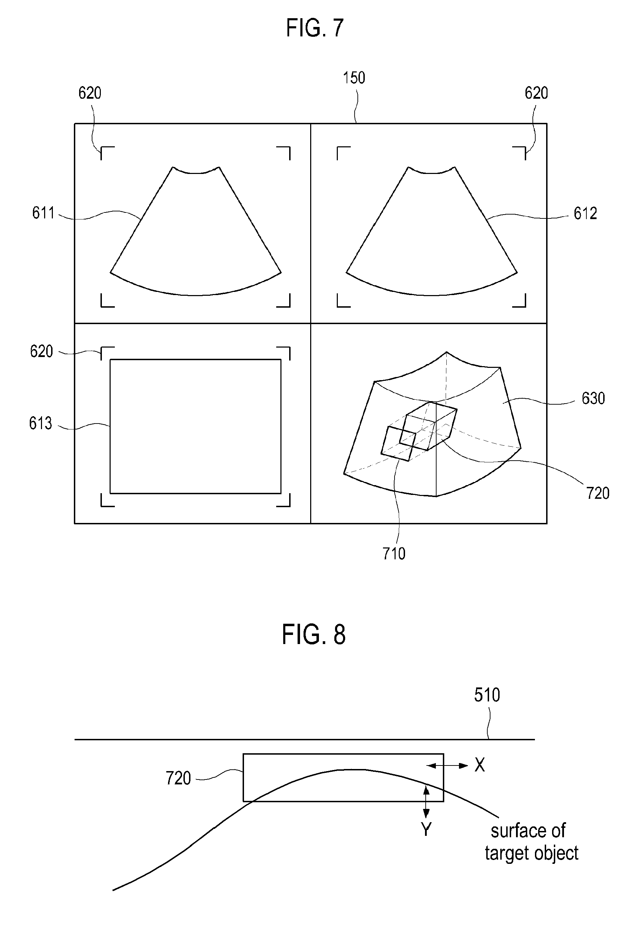

The processing unit 130 may be configured to render volume data corresponding to the ROI to thereby form the 3D ultrasound image, at step S410 in FIG. 4. The methods of rendering the volume data corresponding to the ROI are well known in the art. Thus, they have not been described in detail so as not to unnecessarily obscure the present invention. As one example, the processing unit 130 may render volume data corresponding to the ROI 620 to thereby form the 3D ultrasound image 630 as shown in FIG. 6. The 3D ultrasound image 630 may be displayed on the display unit 150. Thus, the user may define an observation position 710 in the 3D ultrasound image 630, and define a size and a shape of the sub ROI 720 in the 3D ultrasound image 630 based on the observation position 710 as shown in FIG. 7. The observation position 710 may represent a specific part (e.g., a face of a fetus) to observe within the target object.

The processing unit 130 may be configured to perform the image processing upon the 3D ultrasound image in response to the input information (i.e., third input information) provided from the user input unit 120, at step S412 in FIG. 4. The image processing may include a rotation of the 3D ultrasound image, a movement of the 3D ultrasound image and the like. However, it is noted herein that the image processing may not be limited thereto.

The processing unit 130 may be configured to define the sub ROI in the volume data in response to the input information (i.e., second input information) provided from the user input unit 120, at step S414 in FIG. 4.

More particularly, the processing unit 130 may define the observation position 710 in the volume data 510 in response to the input information (i.e., second input information for defining the observation position 710 in the 3D ultrasound image 630 as shown in FIG. 7). The processing unit 130 may further define the sub ROI 720 having a size and a shape in the volume data 510 based on the observation position 710 in response to the input information (i.e., second input information for defining the size and the shape of the sub ROI 720).

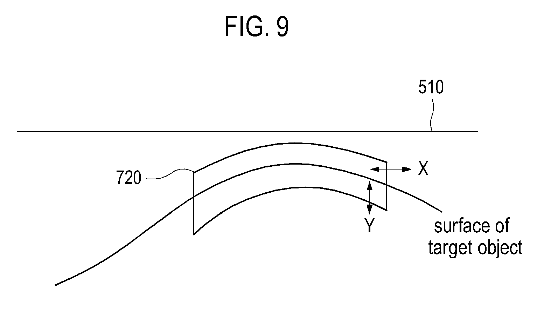

FIGS. 8 and 9 are schematic diagrams showing examples of the sub ROIs. In one embodiment, the processing unit 130 may define the sub ROI 720 having a line shape in the volume data 510, as shown in FIG. 8. In other embodiment, the processing unit 130 may define the sub ROI 720 having a contour shape in the volume data 510, as shown in FIG. 9. It is possible to change the size of the sub ROI 720 in X and Y directions. However, it should be noted herein that the sub ROI may not be limited thereto.

The processing unit 130 may be configured to render volume data corresponding to the sub ROI 720 to thereby form a sub 3D ultrasound image corresponding to the sub ROI 720, at step 5416 in FIG. 4.

Referring back to FIG. 1, the ultrasound system 100 may further include the storage unit 140. The storage unit 140 may store the volume data formed by the processing unit 130. The storage unit 140 may further store the 2D ultrasound images and the 3D ultrasound image formed by the processing unit 130.

The ultrasound system 100 may further include the display unit 150. The display unit 150 may display the plurality of 2D ultrasound images. The display unit 150 may further display the 3D ultrasound image and the sub 3D ultrasound image. In one embodiment, the sub 3D ultrasound image may be displayed on the sub ROI defined in the 3D ultrasound image. In another embodiment, the sub 3D ultrasound image may be displayed with the 2D ultrasound images and the 3D ultrasound images separately.

Although embodiments have been described with reference to a number of illustrative embodiments thereof, it should be understood that numerous other modifications and embodiments can be devised by those skilled in the art that will fall within the scope of the principles of this disclosure. More particularly, numerous variations and modifications are possible in the component parts and/or arrangements of the subject combination arrangement within the scope of the disclosure, the drawings and the appended claims. In addition to variations and modifications in the component parts and/or arrangements, alternative uses will also be apparent to those skilled in the art.

## Claims

1. An ultrasound system, comprising:
an ultrasound data acquisition unit configured to transmit and receive ultrasound signals to and from a target object to output a plurality of ultrasound data;
a user input unit configured to receive first input information and second input information from a user; and
a processing unit in communication with the ultrasound data acquisition unit and the user input unit, the processing unit being configured to form volume data based on the plurality of ultrasound data, form a plurality of 2D ultrasound images corresponding to a plurality of planes based on the volume data, define a region of interest (ROI) in the volume data in response to the first input information for defining the ROI in the plurality of 2D ultrasound images, render volume data corresponding to the ROI to form a 3D ultrasound image, define a sub ROI in the volume data in response to the second input information for defining the sub ROI in the 3D ultrasound image, and render volume data corresponding to the sub ROI to form a sub 3D ultrasound image.

2. The ultrasound system of Claim 1, wherein the processing unit is configured to select the plurality of planes from the volume data.

3. The ultrasound system of Claim 1, wherein the second input information comprises:
information for selecting an observation position from the 3D ultrasound image; and
information for defining a size and a shape of the sub ROI in the 3D ultrasound image.

4. The ultrasound system of Claim 3, wherein the processing unit is configured to:
define the observation position in the volume data in response to the second input information; and
define the sub ROI in the volume data based on the observation position in response to the second input information.

5. The ultrasound system of Claim 1, wherein the user input unit is further configured to receive third input information for performing an image processing upon the 3D ultrasound image.

6. The ultrasound system of Claim 5, wherein the processing unit is further configured to perform the image processing upon the 3D ultrasound image in response to the third input information.

7. The ultrasound system of Claim 6, wherein the image processing comprises at least one of a rotation of the 3D ultrasound image and a movement of the 3D ultrasound image.

8. The ultrasound system of Claim 1, further comprising:
display unit for displaying the sub 3D ultrasound image on the sub ROI defined within the 3D ultrasound image.

9. A method of providing a 3D ultrasound image, comprising:
a) forming volume data based on a plurality of ultrasound data for a target object;
b) forming a plurality of 2D ultrasound images corresponding to a plurality of planes based on the volume data;
c) defining a region of interest (ROI) in the volume data in response to first input information for defining the ROI in the plurality of 2D ultrasound images;
d) rendering volume data corresponding to the ROI to form a 3D ultrasound image;
e) defining a sub ROI in the volume data in response to second input information for defining the sub ROI in the 3D ultrasound image; and
f) rendering volume data corresponding to the sub ROI to form a sub 3D ultrasound image.

10. The method of Claim 9, wherein the step b) is comprises:
selecting the plurality of planes from the volume data;

11. The method of Claim 9, wherein the second input information comprises:
information for selecting an observation position from the 3D ultrasound image; and
information for defining a size and a shape of the sub ROI in the 3D ultrasound image.

12. The method of Claim 11, wherein the step e) comprises:
defining the observation position in the volume data in response to the second input information; and
defining the sub ROI in the volume data based on the observation position in response to the second input information.

13. The method of Claim 9, wherein the step e) further comprises:
receiving third input information for performing an image processing upon the 3D ultrasound image; and
performing the image processing upon the 3D ultrasound image in response to the third input information.

14. The method of Claim 13, wherein the image processing comprises at least one of a rotation of the 3D ultrasound image and a movement of the 3D ultrasound image.

15. The method of Claim 9, further comprising:
i) displaying the sub 3D ultrasound image on the sub ROI defined within the 3D ultrasound image.
